(19) Europäisches Patentamt
European Patent Office
Office européen des brevets

(11) Numéro de publication: **0 097 070**
**A2**

(12) # DEMANDE DE BREVET EUROPEEN

(21) Numéro de dépôt: 83401001.9

(22) Date de dépôt: 19.05.83

(51) Int. Cl.³: **C 07 D 471/04**, C 07 H 19/16, C 07 H 19/20, C 07 C 103/52, A 61 K 31/44, A 61 K 31/70, A 61 K 37/02
// C07H19/06, (C07D471/04, 221/00, 209/00)

(30) Priorité: 24.05.82 FR 8209307

(43) Date de publication de la demande: 28.12.83 Bulletin 83/52

(84) Etats contractants désignés: AT BE CH DE FR GB IT LI LU NL SE

(71) Demandeur: SANOFI, Société dite:, 40, Avenue George V, F-75008 Paris (FR)
Demandeur: Etablissement Public dit: CENTRE NATIONAL DE LA RECHERCHE SCIENTIFIQUE (CNRS), 15, Quai Anatole France, F-75007 Paris (FR)

(72) Inventeur: Auclair, Christian, 54, rue Vavin, F-75006 Paris (FR)
Inventeur: Bernadou, Jean Emile, 23 Rue Devic, F-31400 Toulouse (FR)
Inventeur: Meunier, Gérard, 26 Allée des Jonquilles, F-81600 Gaillac (FR)
Inventeur: Meunier, Bernard, Résidence les Ormes Bat. C3, F-31320 Castanet (FR)
Inventeur: Paoletti, Claude, 84 Rue Michel Ange, F-75016 Paris (FR)
Inventeur: Cier, André, 128 Boulevard Maurice Barrès, F-92200 Neuilly/Seine (FR)

(74) Mandataire: Polus, Camille et al, c/o Cabinet Lavoix 2, Place d'Estienne d'Orves, F-75441 Paris Cedex 09 (FR)

(54) **Procédé de préparation de dérivés de l'ellipticine, nouveaux composés ainsi obtenus et médicament les contenant.**

(57) La présente invention est relative au procédé de préparation dérivés de l'hydroxy-9 ellipticine.

L'invention est également relative aux nouveaux composés ainsi obtenus et à leur application thérapeutique en tant qu'agents anti-tumoreux.

EP 0 097 070 A2

Procédé de préparation de dérivés de l'ellipticine, nouveaux composés ainsi obtenus et médicament les contenant.-

La présente invention est relative à un procédé de préparation de dérivés de l'ellipticine, aux nouveaux composés ainsi obtenus, et à leur application en médecine humaine pour leur activité antitumorale.

Ces composés peuvent aussi être utilisés en tant que dérivés intermédiaires dans la synthèse d'un grand nombre de dérivés employés aussi bien dans l'industrie chimique que pharmaceutique.

L'ellipticine est un composé hétérocyclique dérivé du pyrido-carbazole, possédant des propriétés cytotoxiques et antitumorales (DALTON, L.K., DEMERAC S., ELMES B.C., LEDER J.W., SWAN J.M. et TEITI T., Aust. J. Chem., 1967, 20, 2715), de formule

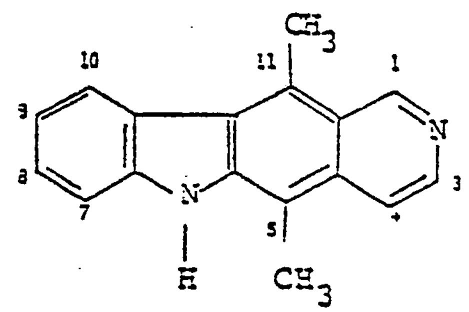

(I)

Le dérivé hydroxylé en position 9 (hydroxy-9 ellipticine) a été préparé. Cependant, si ce dérivé présente une activité antitumorale très supérieure à celle de l'ellipticine sur un grand nombre de tumeurs expérimentales (LE PECQ J.B., DAT XUONG N., GOSSE Ch. et PAOLETTI, Natl. Acad. Sci., USA, 1974, 71, 5078), elle s'est montrée inactive dans le traitement des cancers chez l'homme (LE PECQ, J.B., GOSSE, Ch., DAT XUONG N., CROS S. et PAOLETTI C., Cancer Res., 1976, 36, 3067).

Des recherches effectuées afin d'obtenir un composé utilisable en thérapeutique humaine ont conduit à préparer, à partir de l'hydroxy-9 ellipticine, le dérivé méthylé sur l'azote pyridinique et ont ainsi donné naissance à une série de dérivés de l'ellipticine (LE PECQ J.B., GOSSE Ch., DAT XUONG N., et PAOLETTI C., Comptes-Rendus 1975, 281, série D, 1365), de formule générale

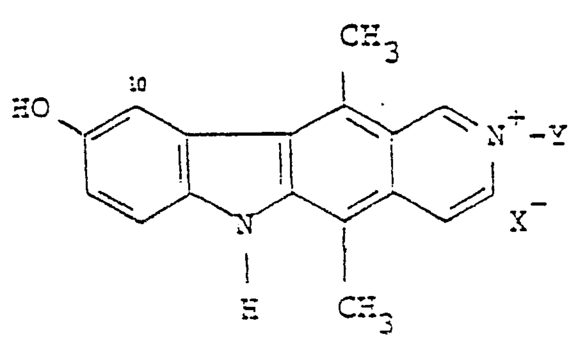

(II)

BAD ORIGINAL

dans lequel Y représente un radical alcoyle inférieur de $C_1$ en $C_4$, hydroxyéthyle ou dialcoylaminoalcoyle et $X^-$ un anion d'acide minéral ou organique pharmaceutiquement acceptable tel que l'acide chlorhydrique, bromhydrique, iodhydrique, sulfurique, phosphorique, hexafluorophosphorique, nitrique, carbonique, citrique, salicylique, méthanesulfonique, acétique, oxalique, malique, fumarique, succinique, aspartique, glutamique, lactique, benzoïque, cyclohexylsulfamique, cinnamique. Un de ces dérivés, l'acétate de méthyl-2 hydroxy-9 ellipticinium est actuellement utilisé avec succès dans le traitement de certains cancers humains et notamment dans le traitement des métastases osseuses des cancers du sein.

La Demanderesse, dans le but de rechercher des composés ayant une plus large diffusion dans les différents compartiments de l'organisme, une meilleure pénétration membranaire, un accroissement de l'affinité pour le DNA, et donc une meilleure et plus large activité antitumorale a étudié les moyens de procéder à la synthèse de nouveaux dérivés de la série ellipticine.

La Demanderesse a découvert à présent un nouveau procédé permettant de procéder à la fixation de substituants sur le carbone 10 des composés de formule (II), ce qui n'avait jamais été réalisé. Elle a, en effet, constaté que dans certaines conditions expérimentales, le carbone 10 des composés de formule (II) était éminemment réactif et formait aisément des liaisons covalentes C-N, C-S, et C-O respectivement avec les acides aminés à l'exception de ceux possédant une fonction -SH libre, avec les acides aminés et les dérivés oligopeptidiques possédant un groupement -SH libre, et avec les composés possédant un groupement -OH libre de la partie ribose d'un nucléoside ou nucléotide naturel.

L'invention a donc pour objet un procédé de préparation de nouveaux composés de la série ellipticine, caractérisé en que le composé de formule (II) ci-dessus, traité par un agent oxydant, se transforme en une quinone-imine très réactive de formule

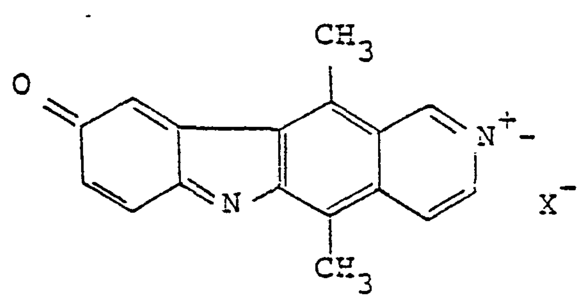

(III)

Cette quinone-imine réagit pour former sur le carbone 10 une liaison C-N avec le groupement $-NH_2$ des acides aminés à l'exclusion de ceux qui possèdent une fonction -SH libre, une liaison C-S avec les acides aminés et les dérivés oligopeptidiques ayant une fonction -SH libre et une liaison C-O avec le groupement OH des ribonucléotides et ribonucléosides.

L'oxydation s'effectue au moyen d'eau oxygénée $H_2O_2$ en présence de peroxydase (Horse Radish Peroxydase ou HRP).

La quinone-imine ainsi obtenue est séparée, puis mise à réagir avec l'acide aminé ou le nucléoside ou nucléotide. La réaction s'effectue en milieu tampon phosphate, à la température ambiante.

Le procédé de l'invention permet en conséquence la préparation de nouvelles ellipticines de formule

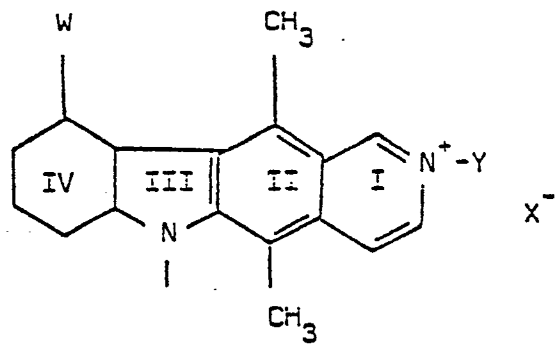 (IV)

dans laquelle Y représente un radical alcoyle inférieur de $C_1$ en $C_4$, hydroxyéthyle ou dialcoylaminoalcoyle et $X^-$ un anion d'acide minéral ou organique pharmaceutiquement acceptable tel que l'acide chlorhydrique, bromhydrique, iodhydrique, sulfurique, phosphorique, hexafluorophosphorique, nitrique, carbonique, citrique, salicylique, méthanesulfonique, acétique, oxalique, malique, fumarique, succinique, ascorbique, glutamique, lactique, benzoïque, cyclohexylsulfamique, cinnamique et W représente

- A : le résidu d'un acide aminé ne présentant pas de groupes -SH libres, de formule

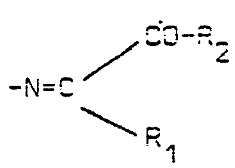

où $R_1$ représente l'hydrogène ou le reste d'un acide aminé et $R_2$ représente l'hydroxyle ou un radical alcoxy inférieur de $C_1$ à $C_4$ ou un reste provenant d'un acide aminé ;

BAD ORIGINAL

- B : le résidu d'un acide aminé ou d'un oligopeptide, présentant un groupe -SH libre, de formule

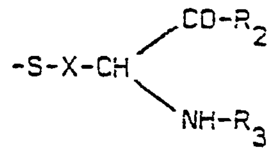

dans lequel X représente un radical hydrocarboné saturé ou non, droit ou ramifié, $R_2$ représente OH ou un radical alcoxy inférieur de $C_1$ à $C_4$ ou un reste provenant d'un acide aminé ou d'un oligopeptide

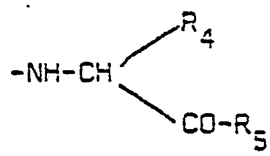

dans lequel $R_4$ représente H ou alcoyle et $R_5$ prend les mêmes valeurs que $R_2$ ; $R_3$ représente l'hydrogène, ou un reste acyle $-OC-R_6$ dans lequel $R_6$ représente un radical alcoyle de $C_1$ à $C_4$ ou $R_3$ représente encore un groupement

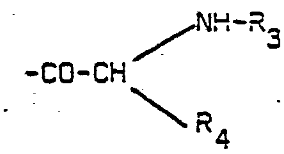

provenant d'un acide aminé ou d'un oligopeptide de formule générale

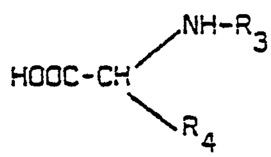

dans laquelle $R_3$ et $R_4$ ont les valeurs définies plus haut,

- C : le résidu d'un nucléoside de formule

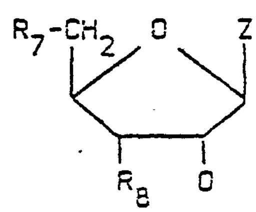

dans laquelle $R_7$ représente OH ou un radical alcoxy inférieur ou

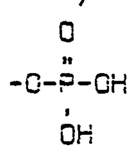 ou 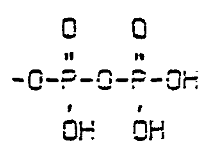 ou 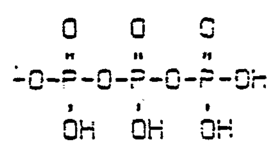

$R_8$ représente OH ou un radical alcoxy inférieur, Z représente l'une des bases azotées de la famille des pyrimidines ou des purines : l'adénine, la guanine, l'uracile, la thymine et la cytosine ; pourvu que lorsque W représente le résidu d'un acide aminé A ou B ou d'un oligopeptide B, les noyaux III et IV aient la structure

BAD ORIGINAL

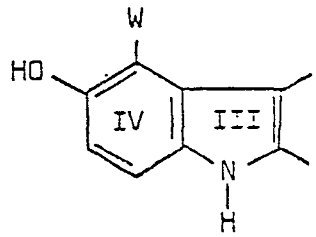

et lorsque W représente le résidu d'un nucléoside C les noyaux III et IV aient la structure

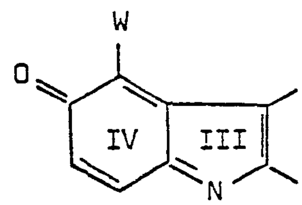

Selon une variante de la méthode décrite ci-dessus, notamment pour les composés à liaison C-N et C-O il n'est pas nécessaire d'isoler la quinone-imine. Ces composés sont, en effet, moins sensibles à l'action de l'agent oxydant, contrairement aux acides aminés et oligopeptides R-SH pour lesquels il est préférable d'éviter toute interaction possible avec le système peroxydasique et donc le risque de formation de composé de formule R-S-S-R.

La réaction s'effectue donc pour ces deux types de composés dans le milieu réactionnel contenant déjà les deux réactifs et la peroxydase, dès que l'eau oxygénée est ajoutée goutte à goutte : la quinone-imine extrêmement réactive, se combine immédiatement, dès sa formation.

Cette réaction de fixation de radicaux sur le carbone 10 est extrêmement sélective. En effet, dans la formation de liaison C-N, l'existence d'hydroxyles OH libres ne vient pas gêner la réaction.

En ce qui concerne les acides aminés et dérivés oligopeptidiques possédant un groupement -SH libre, la création du pont C-S est préférentielle. Il en est de même pour les nucléosides et nucléotides pour la formation du pont C-O : la fixation se fait ici uniquement par l'hydroxyle de la partie ribose.

La présente invention concerne donc aussi les composés préparés par le procédé de l'invention.

Ces composés sont à classer dans 3 séries distinctes :

1/ Les composés à liaison C-N répondant à la formule générale

BAD ORIGIN

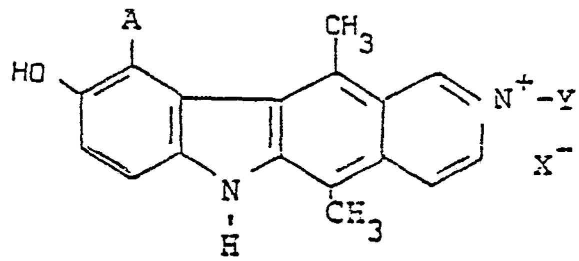

(V)

dans laquelle A représente le groupement

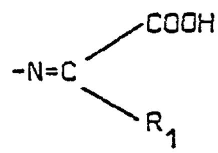

provenant d'un acide aminé de formule générale

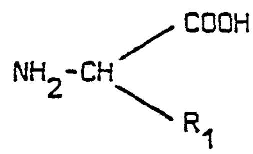

dans lequel $R_1$ peut représenter un radical hydrocarboné saturé ou non, droit ou ramifié, un radical aryle ou aralcoyle, tel que le groupe aryle soit phényle ou hétérocyclique ne devant pas comporter de groupement -SH libre.

2/ Les composés à liaison C-S de formule générale

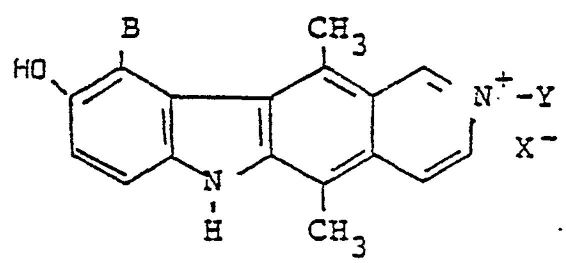

(VI)

dans laquelle B représente le groupement 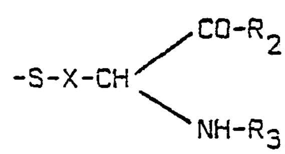

provenant d'un acide aminé ou d'un dérivé oligopeptidique comprenant au plus 10 acides aminés dans lequel X représente un radical hydrocarboné saturé ou non, droit ou ramifié, $R_2$ représente OH ou un radical alcoxy inférieur de $C_1$ à $C_4$ ou un reste provenant d'un acide aminé ou d'un oligopeptide

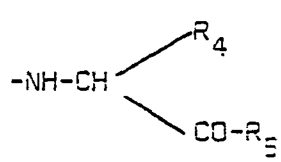

BAD ORIGINAL

dans lequel $R_4$ représente H ou alcoyle et $R_5$ prend les mêmes valeurs que $R_2$ ; $R_3$ représente l'hydrogène, ou un reste acyle $-OC-R_6$ dans lequel $R_6$ représente un radical alcoyle de $C_1$ à $C_4$,
ou $R_3$ représente encore un groupement

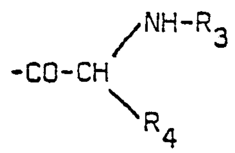

provenant d'un acide aminé ou d'un oligopeptide de formule générale

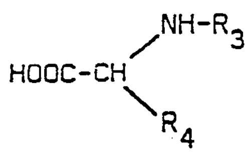

dans laquelle $R_3$ et $R_4$ ont les valeurs définies plus haut.

3/ Les composés à liaison C-O de formule générale

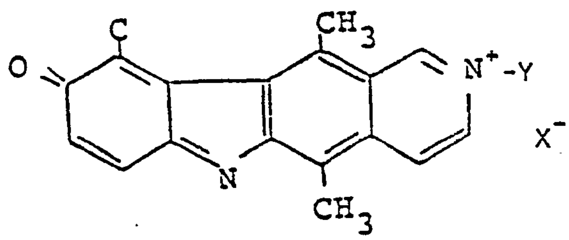

(VII)

dans laquelle C représente un nucléotide ou un nucléoside résultant de l'union du ribose et d'une base purique ou pyrimidinique de formule

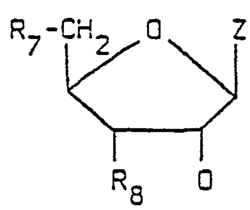

dans laquelle $R_7$ représente OH ou un radical alcoxy inférieur ou

```
    O              O    O              O    O    O
    "              "    "              "    "    "
-O-P-OH    ou   -O-P-O-P-OH    ou   -O-P-O-P-O-P-OH
    '              '    '              '    '    '
    OH             OH   OH             OH   OH   OH
```

$R_8$ représente OH ou un radical alcoxy inférieur,
Z représente l'une des bases azotées de la famille des pyrimidines ou des purines : l'adénine, la guanine, l'uracile, la thymine et la cytosine.

Pour les 3 séries de composés, les radicaux X et Y ont les valeurs définies plus haut.

Les exemples non limitatifs suivants illustrent l'invention.

BAD ORIGINAL

EXEMPLE 1 : acétate de méthyl-2 hydroxy-9 leucine-10, ellipticinium (dérivé n° 1, série C-N)

A = 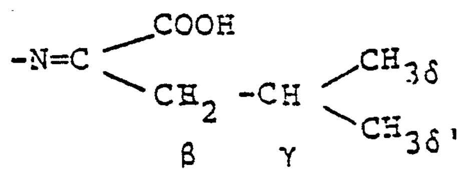

50 mg d'acétate de méthyl-2-hydroxy-9 ellipticinium (1,5 mM) sont dissous dans 25 ml d'eau distillée. D'autre part, 250 mg de leucine sont dissous dans 25 ml d'eau distillée. Après dissolution complète, les deux solutions sont mélangées à 50 ml de tampon phosphate 0,01 M (pH 7,4). Dans la solution obtenue maintenue sous agitation permanente on ajoute 2 mg de peroxydase HRP (Horse Radish Peroxydase). Après dissolution de la peroxydase, on verse goutte à goutte 5 ml d'une solution d'eau oxygénée $H_2O_2$ 40 mM. A la fin de la réaction, la solution est laissée à l'obscurité pendant 30 minutes, puis elle est filtrée.

Toutes ces opérations se sont déroulées à la température ambiante.

La solution est ensuite appliquée sur une colonne hydrophobe biobeads $SM_2$ (3 x 50 cm) préalablement équilibrée avec un tampon acétate d'ammonium 0,1 M (pH 5,4 contenant 1 p. 100 de méthanol).

L'excès d'amino acide ainsi que les éventuels goudrons sont élués par le tampon acétate.

Les composés restant fixés sur la colonne sont ensuite élués par du méthanol pur. La solution éluée est ensuite évaporée au rotavapor à une température inférieur à 40°C. Le résidu obtenu est redissous dans le tampon acétate précédent. Après filtration, la solution est appliquée sur une colonne hydrophobe $XAD_2$ (2,5 x 30 cm) préalablement équilibrée avec le même tampon. On élue ensuite les différentes fractions avec un gradient linéaire, solution de départ : tampon acétate d'ammonium 0,1 M (pH 5,4) contenant 1 p. 100 de méthanol ⟶ 70 p. 100 de méthanol. Le composé recherché est élué en dernière position et il est aisément repérable grâce à sa fluorescence jaune en milieu aqueux.

La solution éluée est ensuite évaporée au rotavapor, le composé est redissous dans l'eau puis lyophylisé.

Rendement : 39 p. 100

Point de fusion : 220°C avec décomposition.

U.V. : solvant $H_2O$

$\lambda$ : 440 nm ($\varepsilon$ = 3000 $M^{-1}$ $cm^{-1}$) ; 340 (3000) ; 300 (32 400) ; 243 (13 750)

BAD ORIGINAL

Masse : masse moléculaire : 463 ; masse cation : 404

DCI ($NH_3$) masse observée : 358

RMN 1H : solvant : $D_2O$ / acétone $d_6$ 50/50 V/V

1,15 (d, 6p, J = 6,4 Hz, 2 $CH_3$ δ et δ' (Leu)) ; 1,93 (s, 3p, OAc) ; 2,37 (m, 1p, $CH_\gamma$ (Leu)) ; 2,66 (s, 3p, $Me_5$) ; 3,00 (d, 2p, J = 6,7 Hz, $CH_{2\beta}$ (Leu)) ; 3,50 (s, 3p, $Me_{11}$) ; 4,48 (s, 3p, $N^+$-Me) ; 7,44 (d, 1p, J = 8,8 Hz, $H_7$ ou $H_8$) ;7,76 (d, 1p, J = 8,8 Hz, $H_7$ ou $H_8$);8,18 (s large, 2p, $H_3$ ou $H_4$) et 9,46(s,1p,$H_1$).

EXEMPLE 2 : acétate de méthyl-2 hydroxy-9 valine 10 ellipticinium (dérivé n° 2, série C-N)

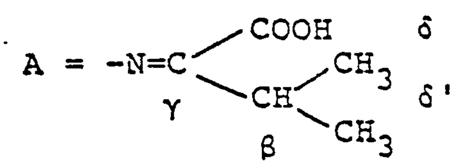

On prépare ce dérivé selon le même mode opératoire à partir de l'acétate de méthyl-2 hydroxy-9 ellipticinium et de la valine.

Rendement : 51 p. 100

Point de fusion : 240°C avec décomposition.

U.V. : solvant $H_2O$

λ : 440 nm (ε = 3 150 $M^1$ $cm^{-1}$) ; 360 (3350) ; 300 (32 850) ; 243 (19 800)

Masse : masse moléculaire : 449 ; masse cation : 390 ;

DCI ($NH_3$) ; masse observée : 344.

RMN 1H : solvant : $D_2O$ / acétone $d_6$ 50/50 V/V

1,58 (d, 6p, J = 60 Hz, 2 $Me_{\gamma\ et \gamma'}$(Val)) ; 1,94 (s, 3p, OAc) ; 2,44 (s, 3p, $Me_5$) ; 3,14 (s, 3p, $Me_{11}$) ; 3,39 (m, 1p, J = 6,8 Hz, $CH_\beta$ (Val)) ; 4,37 (s, 3p, $N^+$-Me) ; 7,17 (d, 1p, J = 8,8 Hz, $H_7$ ou $H_8$) ; 7,52 (d, 1p, J = 8,8 Hz, $H_7$ ou $H_8$), 7,89 (AB, 2p, J = 7,0 Hz, $H_3$ et $H_4$) et 9,12 (s, 1p, $H_1$).

EXEMPLE 3 : acétate de méthyl-2 hydroxy-9 glycine-10 ellipticinium (dérivé n° 3, série C-N)

A = $-N=C_\alpha H-COOH$

On prépare ce dérivé selon le mode opératoire de l'exemple 1 à partir de l'acétate de ményl-2 hycroxy-9 ellipticinium et de la glycine.

Rendement 42 p. 100

BAD ORIGINAL

Point de fusion : supérieur à 250°C

U.V. solvant : $H_2O$

λ = 437 nm (ε = 2550 $M^{-1}$ $cm^{-1}$) ; 360 (3000) ; 300 (31500) 243 (18 360).

Masse : masse moléculaire : 407 ; masse cation : 348 ; DCI ($NH_3$) ; masse observée : 302.

RMN 1H solvant : $D_2O$ / NaOH $d_4$ 50/50 V/V

1,94 (s, 3 p, OAc) ; 2,19 (s, 3p, $Me_5$) ; 2,82 (s, 3p, $Me_{11}$, $Me_{11}$) ; 4,21 (s, 3p, $N^+$-Me) ; 7,00 (d, 1p, J = 8,5 Hz, $H_7$ ou $H_8$) ; 7,48 (d, 1p, J = 8,5 Hz, $H_7$ ou $H_8$) ; 7,56 (d, 1p, J = 7,2 Hz, $H_4$) ; 7,68 (d, 1p, J = 7,2 Hz ; $H_3$) ; 8,34 (s, 1p, $CH_\alpha$ gly) et 8,84 (s, 1p, $H_1$).

<u>EXEMPLE 4</u> : acétate de méthyl-2 hydroxy-9 histidine-10 ellipticinium (dérivé n° 4, série C-N)

A = 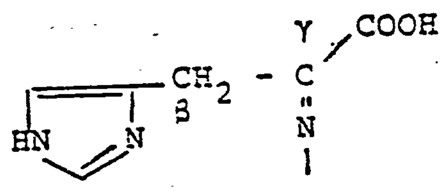

On prépare ce composé selon le mode opératoire de l'exemple 1 à partir de l'acétate de méthyl-2 hydroxy-9 ellipticinium et de l'histidine.

Rendement 41 p. 100

Point de fusion : supérieur à 250°C

U.V. solvant : $H_2O$

λ : 440 nm ( ε = 2750 $M^{-1}$ $cm^{-1}$) ; 360 (6300) ; 305 (24 750) ; 247 (16 950).

Masse : masse moléculaire : 487 ; masse cation : 428 ; DCI ($NH_3$) ; masse observée : 382.

RMN 1H : solvant : $D_2O$ / acétone $d_6$ 30/70 V/V

1,94 (s, 3p, OAc) ; 2, 45 (s, 3p, $Me_5$) ; 3,22 (s, 3p, $Me_{11}$) ; 4,36 (s, 3p, $N^+$-Me) ; 7,20 (d, 1p, J = 9,2 Hz $H_7$ ou $H_8$) ; 7,32 (s, 1p = CH Hist.) ; 7,50 (d, 1p, J = 9,2 Hz, $H_7$ ou $H_8$) ; 7,89 (m, 3p, $H_3$, $H_4$ et =CH Hist.) et 9,26 (s, 1p, $H_1$).

<u>EXEMPLE 5</u> : acétate de méthyl-2 hydroxy-9 alanine-10 ellipticinium (dérivé n° 5, série C-N)

BAD ORIGINAL

A = 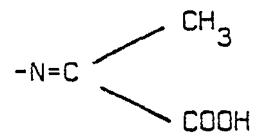

On prépare ce composé selon le mode opératoire de l'exemple 1 à partir de l'acétate de méthyl-2 hydroxy-9 ellipticinium et de l'alanine.

Rendement : 45 p. 100

Point de fusion : 225°C avec décomposition.

U.V. solvant : $H_2O$

$\lambda$ = 440 nm ($\varepsilon$ = 2730 $M^{-1}$ $cm^{-1}$) ; 360 (3000) ; 300 (31050) 243 (16 350).

Masse : masse moléculaire : 421 ; masse cation : 362 ;

DCI ($NH_3$) ; masse observée : 316.

RMN 1H : solvant : $D_2O$ / MeOH $d_4$ 50/50 V/V

1,95 (s, 3p, OAc) ; 2,41 (s, 3p, Me, Ala) ; 2,54 (s, 3p, $Me_5$ ?) ; 3,35 (pic $Me_{11}$ masque par Me OH) ; 4,08 (s, 3p, $N^+$-Me) ; 6,67 (d, 1p, J = 8,0 Hz, $H_7$ ou $H_8$) ; 7,07 (d, 1p, J = 8,0 Hz, $H_7$ ou $H_8$ ; 7,36 (d, 1p, J = 7,0 Hz, $H_3$ ou $H_4$) ; 7,64 (d, 1p, J = 7,0 Hz, $H_3$ ou $H_4$) ; 8,53 (s, 1p, $H_1$).

EXEMPLE 6 : acétate de méthyl-2 hydroxy-9 glutamyle-10 ellipticinium (dérivé n° 6, série C-N)

A = 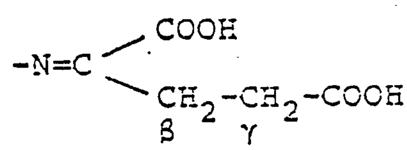

On le prépare selon le mode opératoire décrit à l'exemple 1 à partir de l'acétate de méthyl-2 hydroxy-9 ellipticinium et de l'acide glutamique.

Rendement : 59 p. 100

Point de fusion : supérieur à 250°C.

U.V. solvant : $H_2O$

$\lambda$ = 440 nm ($\varepsilon$ = 2800 $M^{-1}$ $cm^{-1}$) ; 360 (3100) ; 300 (34300) 243 (22 100).

Masse : masse moléculaire : 479 ; masse cation : 420 ;

DCI ($NH_3$) ; masse observée : 374.

BAD ORIGINAL

RMN 1H : solvant $CD_3$ CCOD

2,43 (s, 3p, $Me_5$) ; 3,05 (t, 2p, J = 7,5 Hz ; $CH_{2\gamma}$ glu 3,23 (s large, 5 p, $Me_{11}$ + $CH_{2\beta}$ glu) ; 4,22 (s, 3p, $N^+$-Me) ; 7,18 (d, 1p, J = 8,8 Hz, $H_7$ ou $H_8$) ; 7,41 (d, 1p, J = 8,8 Hz, $H_7$ ou $H_8$) ; 7,72 (AB, 1p, J = 7,4 Hz, $H_3$ et $H_4$) et 9,06 (s, 1p, $H_1$).

EXEMPLE 7 : Hexafluorophosphate de méthyl-2 hydroxy-9 méthylcystéine-10 ellipticinium (dérivé n° 7, série C-S)

B = 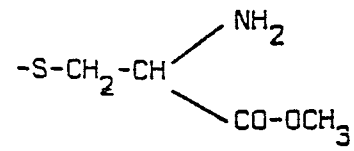

A 500 ml de tampon phosphate à pH 5 (1/15 M) et à température ambiante on ajoute 108 mg de l'acétate de méthyl-2 hydroxy-9 ellipticinium (320 µmoles), 640 µmoles d'eau oxygénée $H_2O_2$ et 5 x $10^{-7}$ moles de HRP. Après 5 minutes d'incubation, on ajoute 3,2 moles d'exafluorophosphate d'ammonium, et la quinone-imine est extraite avec deux litres de chlorure de méthylène ; 948 mg de méthyl-cystéine (7,07 moles) dilués dans 250 ml d'alcool méthylique sont ajoutés à la solution de quinone-imine. Après élimination des solvants à l'évaporateur rotatif, le résidu est repris avec 10 ml de méthanol et précipité avec 450 ml d'éther et 100 ml d'hexane. Une seconde précipitation avec le même mélange de solvants permet d'éliminer ainsi l'excès de méthyl-cystéine. Le méthyl-2 hydroxy-9 ellipticinium est éliminé sur une colonne LH 20 avec le méthanol comme éluant.

Rendement : 34 p. 100

Point de fusion : 220°C avec décomposition.

U.V. solvant : $H_2O$

$\lambda$ = 460 nm ($\varepsilon$ = 2940 $M^{-1}cm^{-1}$) ; 384 (5620) ; 324 (32000) ; 250 (1580) ; 218 (16500).

Masse : masse moléculaire : 554 ; masse cation : 409 ;

DCI ($NH_3$) fragments observés : 323, 309 et 295.

RMN 1H solvant : $D_2O$, $MeOHd_4$ V/V 3 à 1

2,91 (s, 3p, $Me_5$) ; 3,28 (s, 3p, $Me_{11}$) ; 3,47 (d, 2p, J = 5,6 Hz, $-CH_2$-cystéine) ; 3,60 (s, 3p, COOMe cystéine) ; 4,16 (t, 1p, J $\simeq$ 5,0 Hz, -CH-cystéine) ; 4,59 (s, 3p, $N^+$-Me) ; 7,45 (d, 1p, J = 8,8 Hz, $H_7$ ou $H_8$) ; 7,73 (d, 1p, J = 8,3 Hz, $H_7$ ou $H_8$) ; 8,46 (m, 2p, $H_3$ et $H_4$) et 9,96 (s, 1p, $H_1$).

BAD ORIGINAL

EXEMPLE 8 : Hexafluorophosphate de méthyl-2 hydroxy-9 N-acétyl cystéine-10 ellipticinium (dérivé n° 8, série C-S)

B = 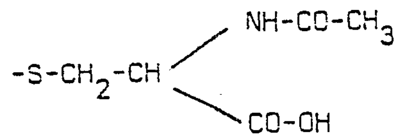

On le prépare avec le mode opératoire décrit à l'exemple 7 à partir de l'acétate de méthyl-2 hydroxy-9 ellipticinium et de la N-acétylcystéine.

Rendement : 36 p. 100

Point de fusion : 240°C avec décomposition.

U.V. solvant : $H_2O$

$\lambda$ = 460 nm ($\varepsilon$ = 3000 $M^{-1}cm^{-1}$) ; 386 (5450) ; 324 (32200) ; 284 (18200) ; 250 (17300).

Masse : masse moléculaire : 583 ; masse cation : 582 ;
(FD) masse observée : 439 ($MH^+$).

RMN 1H solvant : $D_2O$ : acétone $d_6$, V/V = 3 à 1

1,63 (s, 3p, N-acétyl) ; 2,66 (s, 3p, $Me_5$) ; 3,19 (m, 2p, $-CH_2$-cystéine) ; 3,37 (s, 3p, $Me_{11}$) ; 4,09 (m, 1p, -CH-cystéine) ; 4,51 (s, 3p, $N^+$-Me) ; 7,25 (d, 1p, $H_7$ ou $H_8$) ; 7,47 (d, 1p, J = 9,8 Hz, $H_7$ ou $H_8$) ; 8,20 (s large, 2p, $H_3$ et $H_4$) et 9,66 (s, 1p, $H_1$).

EXEMPLE 9 : Hexafluorophosphate de méthyl-2 hydroxy-9 glutathion-10 ellipticinium (dérivé n° 9, série C-S)

B = 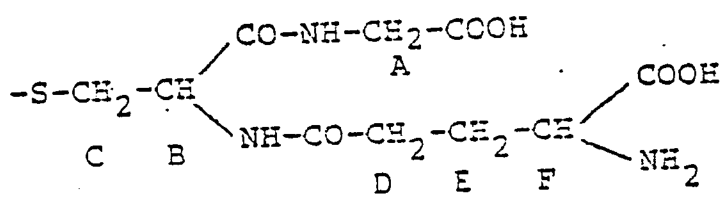

On le prépare avec le mode opératoire décrit à l'exemple 7 à partir de l'acétate de méthyl-2 hydroxy-9 ellipticinium et du glutathion

Rendement : 38 p. 100

Point de fusion 215°C avec décomposition

U.V. solvant : $H_2O$

$\lambda$ = 250 nm ($\varepsilon$ = 18 800 $M^{-1}$ $cm^{-1}$) ; 284 (12300) ; 324 (22 450) ; 386 (4 545 ; 460 (2 090).

Masse : masse moléculaire : 727 ; masse cation : 582,
(FD) masse observée : 583 ($MH^+$)

BAD ORIGINAL

RMN 1H solvant : MeOH $d_4$ (V/V = 3 à 1)

2,05 (m, 2p, (E)) ; 2,14 (s, 3p, $Me_5$) ; 2,40 (m, 2p, (D)) ; 2,71 (s, 3p, $Me_{11}$) ; 2,78 (m, 2p, (C)) ; 3,65 (m, 1p, (F)) ; 3,73 (s, 2p, (A)) ; 4,13 (m, 4p, $N^+$-Me et (B)) ; 6,83 (m, 2p, $H_7$ et $H_8$) ; 7,70 (m, 2p, $H_3$ et $H_4$) ; 9,00 (s, 1p, $H_1$).

A titre d'exemple le spectre RMN du glutathion libre est le suivant : $D_2O$ : Me OH $d_4$ (50/50 V/V)

2,20 (m, 2p, (E)) ; 2,57 (t, 2p; J = 6,8 Hz, (D)) ; 2,92 (d, 2p, J = 6,4 Hz (C)) ; 3,76 (t, 1p, J = 6,2 Hz, (F)) ; 3,95 (s, 2p, (A)) ; 4,58 (t, 1p, 6,4 Hz, (B)).

EXEMPLE 10 : acétate de méthyl-2 oxo-9 adényl-10 ellipticinium (dérivé n° 10, série C-O)

C =

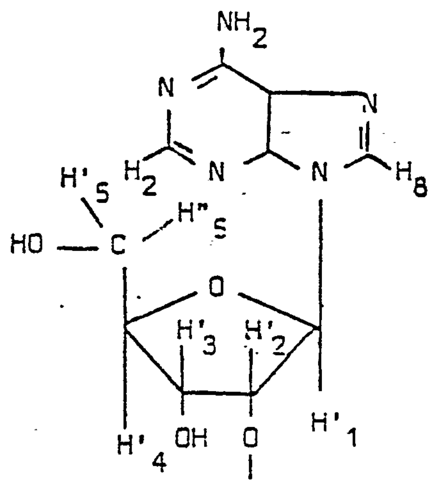

A une solution d'adénosine (71 mg, 0,25 µmole), d'acétate de méthyl-2 hydroxy-9 (17 mg, 0,05 µmole) et 100 µl de HRP $10^{-4}$M dans 20 ml de tampon phosphate 5 x $10^{-2}$ (pH 8), on ajoute en 5 fractions 100 µl d'une solution molaire d'eau oxygénée (0,1 mole). Après 5 minutes d'incubation à température ambiante, le précipité formé est séparé, lavé à l'eau et séché sous vide. Après solubilisation dans 0,5 ml d'alcool méthylique et 0,5 ml d'acide acétique, le produit est précipité avec 4 ml d'éther éthylique, lavé deux fois avec ce même solvant et séché sous vide. On recueille ainsi 15 mg du composé.

Rendement 50 p. 100

Point de fusion > 250°C

U.V. Solvant : $H_2O$

λ = 370 nm (ε = 7000 $M^{-1}$ $cm^{-1}$) ; 315 (13350) ; 272 (36050)

BAD ORIGINAL

Masse : masse moléculaire : 599 ; masse cation : 540 ;
(FD) masse observée : 540
DCI ($NH_3$) masses observées : 528, 407, 291
RMN 1H solvant : $CD_3$ COOD
2,79 (s, 3p, $Me_5$) ; 3,34 (s, 3p, $Me_{11}$) ; 3,85 (m, 2p, $H'_5$et $H''_5$) ; 4,47 (s, 3p, $N^+$-$CH_3$) ; 4,82 (m, 1p, $H'_4$) ; 5,73 (d, 1p, J = 6,2 Hz, $H'_3$) ; 6,05 (d, 1p, J = 6,2 Hz, $H'_2$) ; 6,18 (d, 1p, J = 9,8 Hz, $H_7$) ; 8,16 (d, 1p, J = 7,3 Hz, $H_3$) ; 8,31 (d, 1p, J = 7,3 Hz, $H_4$) ; 8,46 (s, 1p, $H_8$ aden.) ; 8,54 (s, 1p, $H_2$ aden.) et 9,77 (s, 1p, $H_1$).

EXEMPLE 11 : acétate de méthyl-2 oxo-9 guanosyl-10 ellipticinium (dérivé n° 11, série C-O)

R =

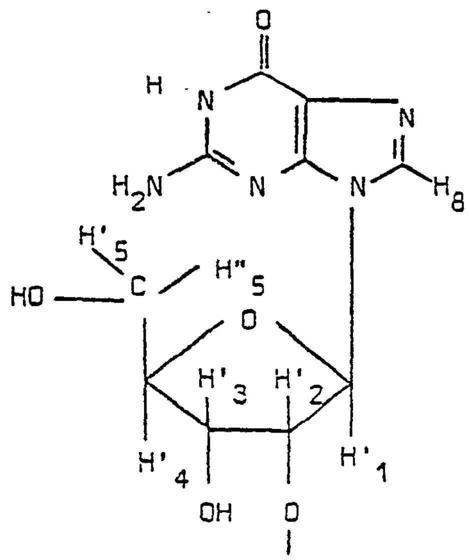

On le prépare avec le mode opératoire décrit à l'exemple 10 à partir de l'acétate de méthyl-2 hydroxy-9 ellipticinium et de la guanosine

Rendement : 81 p. 100
Point de fusion > 250°C
U.V. solvant : $H_2O$
λ = 372 nm (ε = 9350 $M^{-1}cm^{-1}$) ; 315 (20350) 275 (33 450)
Masse : masse moléculaire : 615 ; masse cation : 556 ;
(FD) masse observée 556
DCI ($NH_3$) masses observées : 544, 407, 291.
RMN 1H solvant : $CD_3$ COOD

2,82 (s, 3p, $Me_5$) ; 3,29 (s, 3p, $Me_{11}$) ; 3,79 (m, 2p, $H'_5$ et $H''_5$) ; 4,48 (s, 3p, $N^+-CH_3$) ; 4,70 (m, 1p, $H'_4$) ; 5,69 (d, 1p, J = 7,2 Hz, $H'_3$) ; 6,01 (d, 1p, J = 7,2 Hz, $H'_3$) ; 6,20 (d, 1p, J = 9,8 Hz, $H_8$) ; 6,35 (s, 1p, $H'_1$) ; 7,62 (d, 1p, J = 9,8 Hz, $H_7$) ; 8,15 (s, 1p, $H_8$ guan.) ; 8,17 (d, 1p, $H_3$) ; 8,34 (d, 1p, J = 7, 3 Hz, $H_4$) et 9,75 (s, 1p, $H_1$).

EXEMPLE 12 : acétate de méthyl-2 oxo -9 (adényl -5' -P)-10 ellipticinium (dérivé n° 12, série C-O)

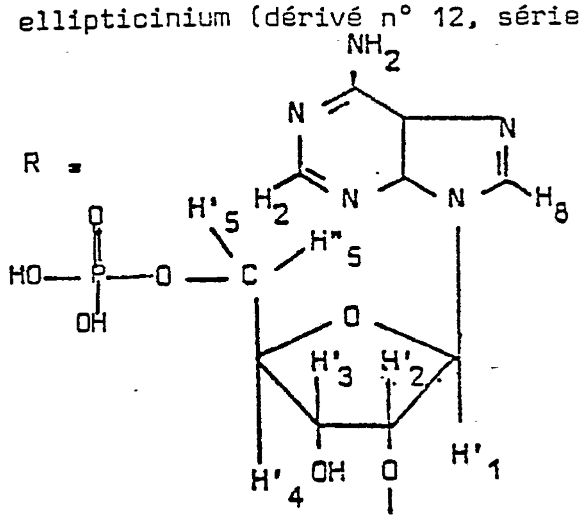

On le prépare avec le mode opératoire décrit à l'exemple 10 à partir de l'acétate de méthyl-2 hydroxy-9 ellipticinium et de l'adénosine monophosphate.

Rendement : 60 p. 100

Point de fusion > 250°C

U.V. Solvant : $CH_3$ COO H /H 20 (50/50)

λ = 440 nm (950 $M^{-1}$ $cm^{-1}$), 372 (4600), 313 (9300) 298 (10 100), 272 (15 100)

Masse : masse moléculaire : 679 ; masse cation : 621

FAB : masse observée : 623

RMN 1H solvant: $CD_3$ COOD : $D_2O$ 50:50 V/V + TMS

2,92 (s, 3H, $Me_5$) ; 3,42 (s, 3H, $Me_{11}$) ; 4,24 (m, 2p, $H'_5$ $H''_5$) ; 4,56 (s, 3H, $N^+-CH_3$) ; 5,11 (m, 1p, $H'_4$) ; (masqué par $H_2O$, $H'_3$) ; 6,24 (d, 1p, J=Hz, $H'_2$) ; 6,37 (d, 1p, J=9,9 Hz, $H_7$) ; 6,73 (s, 1p, $H'_1$) ; 8,13 (d, 1p, J=9,9Hz $H_8$) 8,27 (d, 1p, J=7,3 Hz, $H_3$) ; 8,48 (d, 1p, J=7,3 Hz, $H_4$) ; 8,56 ( s, 1p, $H_8$aden.) ; 8,70 (s, 1p, $H_2$ aden.) ; 9,67 (s, 1p, $H_1$).

BAD ORIGINAL

EXEMPLE 13 : acétate de méthyl -2 oxo -9 (guanylyl -5' -P)-10 ellipticinium (dérivé n° 13, série C-O)

R =

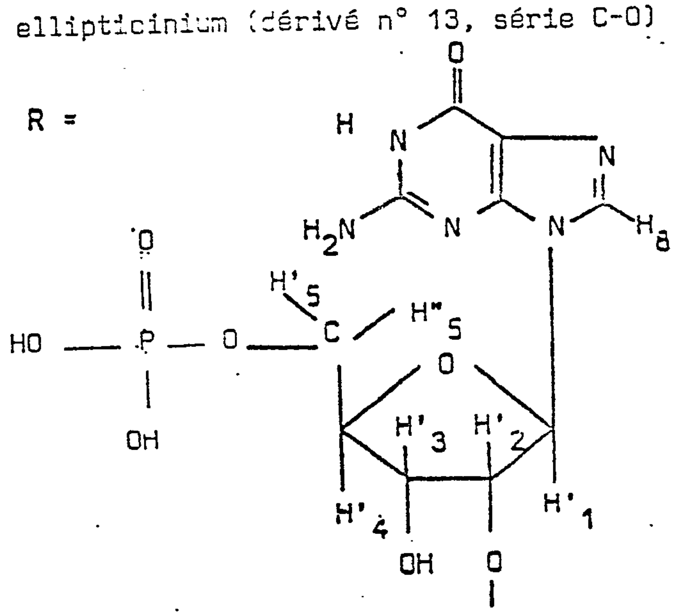

On le prépare avec le mode opératoire décrit à l'exemple 10 à partir de l'acétate de méthyl-2 hydroxy-9 ellipticinium et de la guanosine monophosphate.

Rendement : 41 p. 100

Point de fusion > 250°C

U.V. solvant : $CH_3$ COOH/H2O (50/50)

$\lambda$ = 465 (1900 $M^{-1}$ $cm^{-1}$) ; 370 (10 900) ; 313 (21 000) 300 (24 000) ; 277 (36 600)

Masse : masse moléculaire : 695 ; masse cation : 636

FAB : masse observée : 639

RMN 1H solvant : C $D_3$ COOD/$D_2$O 50:50 V/V + TMS

2,94 (s, 3H, $Me_5$) ; 3,41 (s, 3H, $Me_{11}$) ; 4,31 (m, 2H, $H'_5$ $H''_5$) ; 4,56 (s, 3H, $N^+$-$CH_3$) ; 5,15 (m, 1p, $H'_4$) ; (masqué par $H_2$O $H'_3$) ; 6,16 (d, 1p, 7,2$H_3$, $H'_2$) ; 6,40 (d, 1p, 9,89 $H_3$) ; 6,60 (s, 1p, $H'_1$) ; 7,80 (d, 1p, 9,89 Hz, $H_7$) ; 8,29 (d, 1p, 6,8 Hz, $H_3$) ; 8,50 (d, 1p, 6,8 Hz, $H_4$) ; 9,86 (s, 1H, $H_1$)

L'invention a également pour objet les composés tels qu'ils ont été préparés par le procédé de l'invention.

Les études effectuées ont mis en lumière l'intérêt thérapeutique des composés de l'invention et les résultats des essais toxicologiques et pharmacologiques qui sont rapportés ci-après ont permis de mettre en évidence leur action cytotoxique et antitumorale.

BAD ORIGINAL

L'invention a donc encore pour objet un médicament ayant en particulier des propriétés antitumorales, caractérisé en ce qu'il contient à titre de principe actif, un des dérivés tel que défini ci-dessus.

DETERMINATION DE LA TOXICITE

Cette étude, effectuée sur des souris femelles de type DBA/2 d'un poids corporel moyen de 20 g a permis de déterminer la $DL_0$, qui représente la dose unique maximum administrable par une injection intrapéritonéale sans qu'aucune mort intervienne dans les 30 jours qui suivent l'administration du produit.

Les résultats sont consignés dans le tableau suivant :

| DERIVES | $DL_0$ en mg / Kg |
|---|---|
| n° 1 | 10 |
| n° 2 | 15 |
| n° 3 | 20 |
| n° 5 | 20 |

ETUDE DE LA CYTOTOXICITE

L'activité inhibitrice des composés de l'invention sur la croissance des cellules tumorales L 1210 en culture est déterminée par l'estimation de la $ID_{50}$ obtenue après 48 heures de culture en présence de doses croissantes de drogue.

Le $ID_{50}$ correspond à la quantité de produit nécessaire à l'obtention d'une inhibition de 50 p. 100 de la multiplication cellulaire.

Les cellules leucémiques L 1210 adaptées à la culture en suspension stationnaire et exemptes de contamination bactérienne ou mycoplasmique (M.F. BARILE, in : J. FOGH Ed., Contamination in tissue culture, Academic Press, New-York, 1973, P. 132), sont ensemencées, après numération, à la concentration de $1 \times 10^5$ cellules / ml et cultivées par 2 ml dans des flacons de pharmacie de 15 ml. Les substances à étudier sont dissoutes dans du DMSO (1 p. 100 final) dont on a préalablement vérifié l'innocuité. Des quantités croissantes du produit à tester sont ajoutées au temps 0 et des numérations sont faites après 24 heures et 48 heures de contact. Des séries témoins sont constituées avec des quantités de DMSO équivalentes à celles des séries traitées. Le temps de doublement du témoin est de 12 heures environ.

Le pourcentage d'inhibition de la croissance cellulaire est déterminé par le rapport de la pente de la courbe de croissance du témoin sur la pente de la courbe de croissance des essais pour différentes concentrations de produit à tester après 24 heures et 48 heures de culture.

Une relation dose effet est déterminée selon la méthode graphique de LICHTFIELD et WILCOXON (C. DUPONT, J. Pharmacol. Paris, 1970, 1, 3, 407-414), d'après les résultats obtenus. La concentration de substance qui diminue la croissance cellulaire de 50 p. 100 $ID_{50}$ est déterminée d'après les droites obtenues.

Les résultats sont consignés dans le tableau suivant :

| DERIVES | $ID_{50}$ en uM |
|---|---|
| n° 1 | 0,26 |
| n° 2 | 0,31 |
| n° 3 | 0,54 |
| n° 4 | 2,46 |
| n° 5 | 0,20 |
| n° 6 | 23,8 |
| n° 7 | 0,79 |
| n° 8 | 1,12 |
| n° 9 | 1,81 |
| n°10 | 0,50 |
| n°11 | 0,78 |
| n°12 | > 0,80 |
| n°13 | > 1,00 |

BAD ORIGINAL

MESURE DE L'EFFET ANTITUMORAL

Les cellules tumorales L 1210 sont inoculées par la voie intrapéritonéale à des souris femelles DBA/2 d'un poids corporel moyen de 20g en une injection unique de $10^5$ cellules; chez les animaux traités, le composé étudié est injecté à la dose de $DL_0/2$ également par la voie intrapéritonéale 20 heures après la première injection des cellules L 1210.

L'effet antitumoral est apprécié par la détermination de l'augmentation de la durée de survie (ILS : increase in life Span) des animaux traités par rapport aux animaux témoins qui n'ont reçu que les cellules tumorales.

L'augmentation de la durée de survie est considérée comme significative lorsqu'elle est supérieure à 25 p. 100.

Les résultats obtenus sont consignés dans le tableau suivant qui indique pour les dérivés testés, le pourcentage d'augmentation de la durée de survie par rapport au témoin.

| DERIVES | POURCENTAGE D'AUGMENTATION DE LA DUREE DE SURVIE (ILS) |
|---|---|
| n° 1 | 35 |
| n° 2 | 31,3 |

Les études qui viennent d'être rapportées ont mis en évidence les intéressantes propriétés antitumorales des composés de l'invention qui les rendent très utiles en thérapeutique humaine.

Le médicament de l'invention pourra être administré par les voies orale, intramusculaire ou intraveineuse, sous forme de comprimés, comprimés dragéifiés, capsules, poudre ou soluté injectable.

Chaque dose unitaire contient avantageusement de 5 mg à 500 mg de principe actif en association avec des excipients et véhicules pharmaceutiquement acceptables ; les doses administrables journellement étant fonction de l'ampleur, de la rapidité et du degré d'efficacité recherchés pourront varier de 1 mg à 250 mg/kg de poids corporel.

REVENDICATIONS

1. Procédé de préparation de nouveaux dérivés de l'ellipticine caractérisé en ce qu'on traite un sel d'alcoyle-2 hydroxy-9 ellipticinium de formule

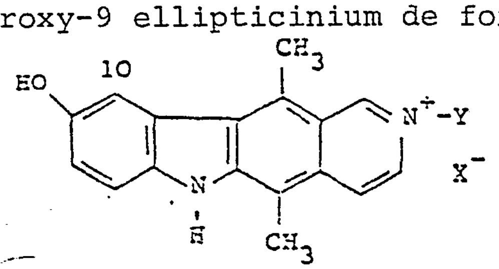

(II)

dans lequel

Y représente un radical alcoyle inférieur de $C_1$ à $C_4$, hydroxyéthyle ou dialcoylaminoalcoyle et $X^-$ est un anion d'acide minéral ou organique pharmaceutiquement acceptable tel que l'acide chlorhydrique, bromhydrique, iodhydrique, sulfurique, phosphorique, hexafluorophosphorique, nitrique, carbonique, citrique, salicylique, méthane-sulfonique, acétique, oxalique, malique, fumarique, succinique, aspartique, glutamique, lactique, benzoïque, cyclohexylsulfamique , cinnamique, par un agent oxydant pour le transformer en une quinone-imine de formule

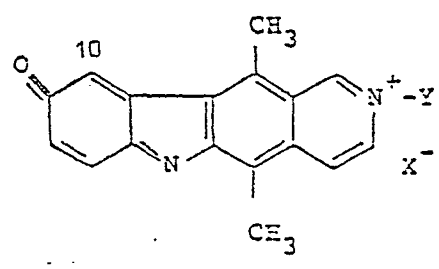

(III)

que l'on fait réagir avec les acides aminés à l'exception de ceux possédant une fonction -SH libre pour obtenir sur le carbone 10 une liaison C-N, avec les acides aminés et les oligopeptides comprenant au plus 10 acides aminés, et possédant une fonction -SH libre,

pour obtenir sur le carbone 10 une liaison C-S ou avec les ribonucléotides et ribonucléosides possédant un groupement -OH libre de la partie ribose d'un nucléotide ou nucléoside naturel,pour obtenir sur le carbone 10 une liaison C-O.

2. Procédé selon la revendication 1 caractérisé en ce que le composé de formule (II) est oxydé en quinone-imine de formule (IV) puis isolé du milieu réactionnel.

3. Procédé selon la revendication 2 caractérisé en ce que l'oxydation est effectuée par l'eau oxygénée en présence d'une peroxydase.

4. Procédé selon l'une des revendications 2 et 3, caractérisé en ce que la réaction d'oxydation s'effectue en milieu tampon-phosphate à la température ambiante.

5. Procédé selon l'une des revendications 1 à 4 caractérisé en ce que, selon une variante, la quinone-imine n'est pas isolée du milieu réactionnel dans le cas où la réaction sur le carbone 10 s'effectue avec des groupements $-NH_2$ ou -OH.

6. Dérivés de l'ellipticine à liaison C-N caractérisés en ce qu'ils répondent à la formule générale

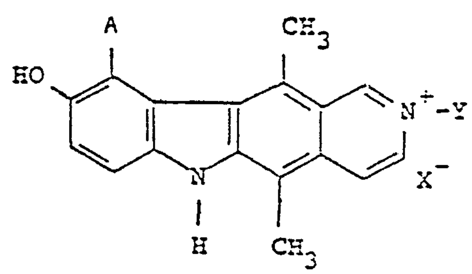

(V)

dans laquelle A représente le groupement 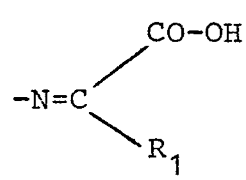

provenant d'un acide aminé de formule générale

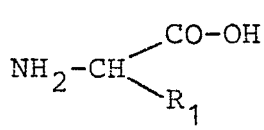

dans lequel $R_1$ représente un radical hydrocarboné saturé ou non, droit ou ramifié, un radical aryle ou aralcoyle, tel que le groupe aryle soit phényle ou hétérocyclique ne devant pas comporter de groupement -SH libre ; Y représente un radical alcoyle inférieur de $C_1$ à $C_4$, hydroxyéthyle ou dialcoylaminoalcoyle et $X^-$ est un anion d'acide minéral ou organique pharmaceutiquement acceptable tel que l'acide chlorhydrique, bromhydrique, iodhydrique, sulfurique, phosphorique, hexafluorophosphorique, nitrique, carbonique, citrique, salicylique, méthane-sulfonique, acétique, oxalique, malique, fumarique, succinique, aspartique, glutamique, lactique, benzoique, cyclohexylsulfamique, cinnamique.

7. Dérivés de l'ellipticine à liaison C-S caractérisés en ce qu'ils répondent à la formule générale

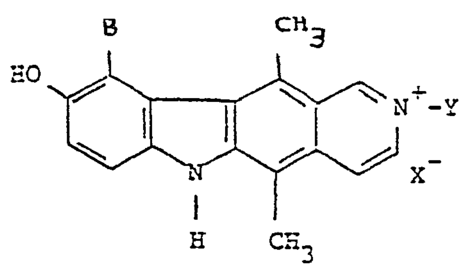

(VI)

dans laquelle B représente le groupement 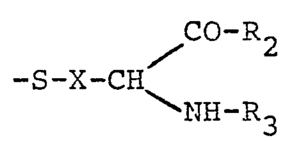

provenant d'un acide aminé ou d'un dérivé oligopeptidique comprenant au plus 10 acides aminés dans lequel X représente un radical hydrocarboné saturé ou non, droit ou ramifié, $R_2$ représente -OH ou un radical alcoxy inférieur de $C_1$ à $C_4$ ou un reste provenant d'un acide aminé ou d'un oligopeptide

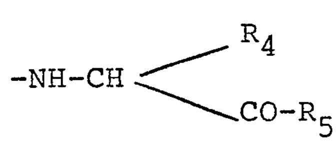

dans lequel $R_4$ représente H ou un groupe alcoyle et $R_5$ a les mêmes valeurs que $R_2$ ; $R_3$ représente l'hydrogène, ou un reste acyle $-OC-R_6$ dans lequel $R_6$ représente un radical alcoyle de $C_1$ à $C_4$ ou $R_3$ représente encore un groupement

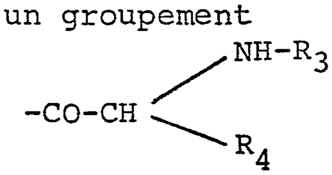

provenant d'un acide aminé ou d'un oligopeptide de formule générale

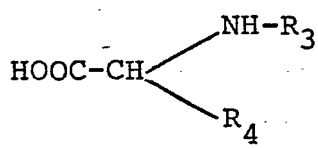

dans laquelle $R_3$ et $R_4$ ont les valeurs définies plus haut, et X et Y sont tels que définis dans la revendication 6.

8. Dérivés de l'ellipticine à liaison C-O, caractérisés en ce qu'ils répondent à la formule générale

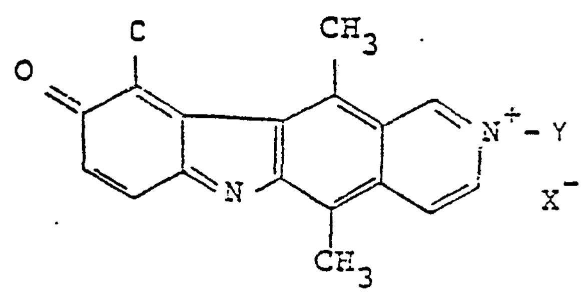

(VII)

dans laquelle C représente un nucléotide ou un nucléoside résultant de l'union du ribose et d'une base purique ou pyrimidinique, de formule

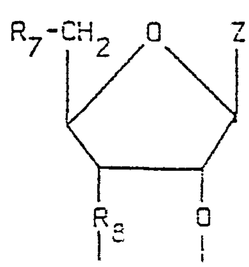

dans laquelle $R_7$ représente OH ou un radical alcoxy inférieur ou

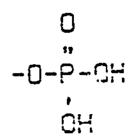 ou 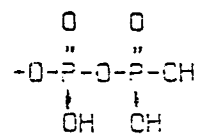 ou 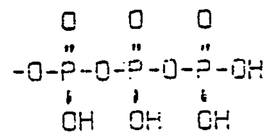

$R_8$ représente OH ou un radical alcoxy inférieur, Z représente l'une des bases azotées suivantes de la famille des pyrimidines ou des purines : l'adénine, la guanine, l'uracile, la thymine et la cytosine et X et Y sont tels que définis dans la revendication 6.

9. Acétate de méthyl-2 hydroxy-9 leucine-10 ellipticinium.

10. Acétate de méthyl-2 hydroxy-9 valine -10 ellipticinium.

11. Acétate de méthyl-2 hydroxy-9 glycine-10 ellipticinium.

12. Acétate de méthyl-2 hydroxy-9 histidine-10 ellipticinium.

13. Acétate de méthyl-2 hydroxy-9 alanine-10 ellipticinium.

14. Acétate de méthyl-2 hydroxy-9 gultamyle-10 ellipticinium.

15. Hexafluorophosphate de méthyl-2 hydroxy-9 méthylcystéine-10 ellipticinium.

16. Hexafluorophosphate de méthyl-2 hydroxy-9 N-acétyl cystéine-10 ellipticinium.

17. Hexafluorophosphate de méthyl-2 hydroxy-9 glutathion-10 ellipticinium.

18. Acétate de méthyl-2 oxo-9 adényl-10 ellipticinium.

19. Acétate de méthyl-2 oxo-9 guanosyl-10 ellipticinium.

20. Acétate de méthyl -2 oxo-9 (adényl-5'-P)-10 ellipticinium.

21. Acétate de méthyl -2 oxo-9 (guanylil-5'-P) -10 ellipticinium.

22. Médicament ayant notamment une action antitumorale caractérisé en ce qu'il contient à titre de principe actif un composé selon l'une des revendications 6 à 21.

23. Médicament selon la revendication 22, caractérisé en ce qu'il est présenté sous une forme appropriée à l'administration orale ou parentérale.

24. Médicament selon l'une des revendications 22 et 23 caractérisé en ce qu'il est présenté sous forme de doses unitaires contenant de 5 mg à 500 mg de principe actif.